# EUROPÄISCHE PATENTSCHRIFT

(11) **EP 1 398 046 B1**
(45) Veröffentlichungstag und Bekanntmachung des Hinweises auf die Patenterteilung: **27.07.2005**
(21) Anmeldenummer: 03019738.8
(22) Anmeldetag: 29.08.2003
(51) Int. Cl.: A61L 27/34, A61L 27/54, A61L 29/08, A61L 29/16, A61L 31/10, A61L 31/16

(54) **Antibiotikum-/Antibiotika-Polymer-Komposit**
Antibioticum-/Antibiotica-polymer composite
Composite antibiotique(s)-polymère

(30) Priorität: 11.09.2002 DE 10242476
(43) Veröffentlichungstag der Anmeldung: 17.03.2004
(73) Patentinhaber: Heraeus Kulzer GmbH, 63450 Hanau (DE)
(72) Erfinder: Vogt, Sebastian, Dr., 99084 Erfurt (DE); Schnabelrauch, Matthias, Dr., 07749 Jena (DE); Kühn, Klaus-Dieter, Dr., 35041 Marburg (DE)
(74) Vertreter: Kühn, Hans-Christian

(56) Entgegenhaltungen:
- EP-A- 0 372 130
- EP-A- 0 879 595
- US-A- 5 451 424

## Beschreibung

Die vorliegende Erfindung betrifft eine Antibiotikum-/Antibiotikum-Polymer-Kombination, die unter physiologischen Bedingungen eine kontinuierliche Freisetzung von Antibiotika über einen Zeitraum von mehreren Tagen gewährleistet und in der Human- und Veterinärmedizin eingesetzt werden kann.

In der Human- und Veterinärmedizin werden Medizinprodukte aus Kunststoffen in Form von Drainagen, Kathetern, Abdeckfolien und Netzen als temporäre oder dauerhafte Implantate zur Sekretabsaugung, Spülung, Abdeckung und Fixierung eingesetzt. Problematisch ist hierbei, dass Mikroorganismen insbesondere bei Drainagen und Kathetern entlang dieser Kunststoffschläuche in den Organismus einwandern können und dadurch lokale Infektionen verursachen, die sich unbehandelt weiter im Organismus ausbreiten können. Ähnliche Probleme treten beim Einsatz von Fixateuren externe auf. Dabei können Mikroorganismen in gleicher Weise entlang der Verstiftungen in den Organismus eindringen. Auch bei Dentalimplantaten sind Infektionsprobleme an den Implantatoberflächen bekannt. Hieraus ergibt sich die Notwendigkeit, bei der medizinischen Anwendung dieser Implantate eine Infektionsprophylaxe bzw. eine Infektionsbekämpfung vorzunehmen. Diese Infektionsunterdrückung kann grundsätzlich systemisch oder lokal mit geeigneten Antibiotika erfolgen. Die systemische Anwendung von Antibiotika ist mit einer Reihe von Problemen behaftet. Um antimikrobiell-wirksame Antibiotika-Konzentrationen systemisch erreichen zu können, sind relativ hohe Antibiotika-Dosen erforderlich. Dadurch kann es insbesondere bei den Antibiotika des Aminoglykosid-Typs und bei den Antibiotika des Tetracyclin-Typs zu unerwünschten Schädigungen aufgrund ihrer Nephrotoxizität bzw. Ototoxizität kommen. Daher ist eine Infektionsunterdrückung durch lokale Anwendung von Antibiotika sinnvoller, weil hierbei wirksame lokale Antibiotika-Konzentrationen erreicht werden können, unter Vermeidung von hohen systemischen Antibiotika-Konzentrationen.

Die Herstellung und Verwendung von antibiotischen Polymer-Kompositen ist seit Jahren Gegenstand intensiver Forschungen, die zu einer Reihe von Patenten führten. So legten Shepherd und Gould eine Beschichtung von Kathetern mit hydrophilen Polymethacrylaten und Polyacrylaten offen, in die ein nicht näher spezifiziertes Antibiotikum zur Behandlung von Infektionen eingebracht ist (T. H. Shepherd, F. E. Gould: Catheter. 03.03.1971, US 3,566,874). Ebenfalls von Shepherd und Gould stammt ein in den 1970er Jahren beschriebenes Retardsystem auf Basis von hydrophilen Hydroxyalkylacrylaten und Hydroxymethacrylaten, die zu antibiotisch ausgerüsteten Formkörpern polymerisiert werden (T. H. Shepherd, F. E. Gould: Dry hydrophilic acrylate or methacrylate polymer prolonged release drug implants. 31.12.1974, US 3,857,932). Klemm beschrieb aus Polymethacrylat und Polyacrylat aufgebaute Kunststoffpartikel zur Behandlung von Osteomyelitis (K.Klemm: surgical synthetic-resin material and method of treating osteomyelitis. 13.05.1975, US 3,882,858). Diese Kunststoffpartikel waren mit Gentamicin oder einem anderen Antibiotikum impregniert. Von Gross et al. stammt ein fortgeschrittener Vorschlag zur Herstellung von Knochenzement, der Gentamicin enthält (A. Gross, R. Schaefer, S. Reiss: Bone cement compositions containing gentamycin. 22.11.1977, US 4,059,684). Hierbei werden als Hilfsstoffe in Wasser leicht lösliche Salze wie Natriumchlorid, Kaliumchlorid, Natriumbromid und Kaliumbromid zu einem Gemisch bestehend aus gepulverten Kopolymeren von Methylmethacrylat und Methylacrylat, Methylmethacrylat, Gentamicinhydrochlorid und/oder Gentamicinsulfat gegeben. Diese Mischung wurde durch Peroxide polymerisiert. Die in Wasser leicht löslichen Salze lösen sich nach Einbringung des Knochenzementes in physiologisches Milieu und hinterlassen Hohlräume. Von Batich et al. wurde ein neues Freisetzungssystem auf Grundlage von Kopolymeren beschrieben, das unter Verwendung von schwach sauren Monomeren synthetisiert wurde, und das ab einem pH-Wert von 8,5 quillt und dadurch eine Wirkstofffreisetzung von eingeschlossenen pharmazeutischen Wirkstoffen ermöglichen soll (C D. Batich, M. S. Cohen, K. Forster: Compositions and devices für controlled release of active ingredients. 10.10.1996, US 5,554,147).

Der Gegenstand einer Reihe weiterer Arbeiten war die antimikrobielle Beschichtung von Medizinprodukten mit antibiotischen Polymersystemen. So entwickelten Conway et al. eine Polymermatrix aus Silicon, in die in Wasser lösliche Wirkstoffe auf Nitrofuran-Basis fein verteilt eingeschlossen wurden (A. J. Conway, P. J. Conway, R.D. Fryar Jr.: Sustained release bactericidal cannula. 16.11.1993, US 5,261,896). Die Verwendung eines Matrix-bildenden Polymers aus der Gruppe der Polyurethane, der Silicone und der bioabbaubaren Polymere, in denen ein Gemisch aus einem Silbersalz und Chlorhexidin suspendiert ist, wurde zur Herstellung von Infektions-resistenten Medizinprodukten offengelegt (C. L. Fox Jr., S. M. Modak, L. A. Sampath: Infection-resistant compositions, medical devices and surfaces and methods for preparing and using same. 28.05.19991, US 5,019,096). Von Solomon, Byron und Parke wurden ähnliche antiinfektive Systeme auf Basis von Polyurethan und darin dispergiertem Chlorhexidin vorgeschlagen (D. D. Solomon, M P. Byron: Anti-infective and antithrombogenic medical articles and method for their preparation. 19.09.1995, US 5,451,424; D. D. Soloman, M. P. Parke: Anti-infective and antithrombogenic medical articles and method for their preparation. 13.01.1998, US 5,707,366; D. D. Soloman, M. P. Parke: Anti-infective and antithrombogenic medical articles and method for their preparation. 13.01.1998, US 5,165,952). Diese Systeme konnten aus der Schmelze durch Extrusion zu Formkörpern verarbeitet werden. Eine antibiotische Komposition, die aus oligodynamisch wirkenden Metallen und Polymeren zusammengesetzt ist, wurde ebenfalls offengelegt (D. Laurin, J. Stupar: Antimicrobial compositions. 29.07.1984, US 4,603,152). Als Polymere werden Acrylnitril-Butadien-Styren-Kopolymere, Polyvinylchlorid, Polyester, Polyurethane, Styren-Block-Kopolymere und Gummi vorgeschlagen in denen oligodynamisch wirksame Metalle zur Infektionsunterdrückung eingebracht sind. Auch Elastomere konnten antibiotisch ausgerüstet werden. So erzeugte Allen Elastomer-Wirkstoffkombinationen durch Hinzumischung und Einarbeitung von Wirkstoffen in Gummi-Masterbatchs (D. L. Allen: Elastomeric composition containing therapeutic agents and articles manufactured therefrom. 28.05.1991, US 5,019,378). Die Masterbatchs setzten sich aus Gummi, Glimmer und Titandioxid zusammen. Eine antibiotische Beschichtung bestehend aus einem Gemisch von Rifampin und Minocyclin, die in einem Polymer dispergiert wurden, schlagen Raad und Darouiche vor (I. I. Raad, R. O. Darouiche: Antibacterial coated medical implants. 08.06.1993, US 5,217,493). Das Polymermaterial ist hierbei nicht näher charakterisiert. De Leon et al. legen eine Methode zur antibiotischen Beschichtung von Implantaten offen, bei denen die zu beschichtende Oberfläche zuerst mit Siliconöl beschichtet wird (J. De Leon, T. H. Ferguson, D. S. Skinner Jr.: Method of making antimicrobial coated implants. 28.03.1990, US 4,952,419). Auf die Siliconöl-Schicht bringt man in einem zweiten Schritt den gepulverten Wirkstoff auf. Oxytetracyclin wurde hierbei als Wirkstoff verwendet. Eine ähnliche Beschichtung auf Basis von Silicon-Öl und Poly(methacrylsäureester) wurden von Takigawa beschrieben, die ausgehend von einer Lösung von Silicon-Öl und Poly(methacrylsäureester) in Terpentin-Öl, N-Decane, Tetrachlormethan, Butan-2-on, 1,4-Dioxan, Ethoxyethanol und Toluen hergestellt wurde (B. Takigawa: Coating solution containing silicone oil and polymethacrylate. 24.1998, US 5,721,301). Mustacich et al. beschreiben eine antimikrobielle Polymer-Kombination, bei der Fettsäuren und Fettsäuresalze als biozide Reagenzien in medizinisch anwendbare Polymere eingebracht werden (R.V. Mustacich, D. S. Lucas, R. L. Stone: Antimicrobial polymer compositions. 30.10.1984, US 4,479,795).

Eine interessante Beschichtungs-Komposition wurde von Whitboume und Mangan offengelegt, bei der quartäre Ammoniumverbindungen als antimikrobielles Reagens in ein Wasser unlösliches Polymer, zum Beispiel in Celluloseester, eingearbeitet sind (R. J. Whitboume, M. A. Mangan: Coating compositions comprising pharmaceutical agents. 11.06.1996, US 5,525,348). Von Friedmann et al. wurden eine Reihe von Patenten bekannt, die sich mit der Herstellung von Dentallacken befassen (M. Friedmann, D. Steinerg, A.Soskolne: Sustained-release pharmaceutical compositions. 11.06.1991, US 5,023,082; M. Friedman, A. Sintov: Liquid polymer composition, and method of use. 03.11.1992, US 5,160,737; M. Friedman, A. Sintov: Dental vamish composition, and method of use. 19.07.1994, US 5,330,746; M. Friedman, A. Sintov: Dental vamish composition and method of use. 15.07.1997, US 5,648,399; M. Friedman, A. Sintov: Dental vamish composition, and method of use.17.06.1997, US 5,639,795). Diese Patente sind inhaltlich nahezu gleich und enthalten als wesentliche antimikrobielle Substanzen quartemäre Ammoniumsalze. Es werden in den Patenten Lacke und Polymerlösungen zu deren Herstellung beschrieben, die im wesentlichen aus folgenden Komponenten bestehen: einem aus Methacrylsäure und Methacrylsäureestem aufgebauten Kopolymeren mit freien Carbonsäuregruppen, einem aus Methacrylsäure und Methacrylsäuremethylester aufgebauten Kopolymeren mit freien Carbonsäuregruppen, einem aus Dimethylaminoethylacrylat und Ethylmethacrylat aufgebauten Kopolymeren und einem aus Methylacrylat und Chlortrimethylammoniumethylmethacrylat gebildeten Kopolymer. Bei US 5,648,399 ist interessant, dass ein die Wirkstofffreisetzung beeinflussendes Reagenz aus der Gruppe der quervemetzenden Reagenzien, der Polysaccharide, der Lipide, der Polyhydroxyverbindungen, der Polycarbonsäuren, divalente Kationen, Zitronensäure, Natriumzitrat, Natriumdocusat, Proteine, Polyoxyethylensorbitanmonooleat und Aminosäuren der Polymer-Kombination zugesetzt wird.

Von Bayston und Grove stammt ein interessanter Vorschlag zur Herstellung von antimikrobiellen Medizinprodukten (R. Bayston, N. J. Grove: Antimicrobial device and method. 17.04.1990, US 4,917,686). Hierbei werden antibiotische Substanzen in einem geeigneten organischen Lösungsmittel gelöst. Diese Lösung lässt man auf die zu modifizierenden Polymeroberflächen einwirken. Durch das Lösungsmittel quillt das Polymer an und der Wirkstoff kann in die Oberfläche eindringen. Darouiche und Raad schlagen eine prinzipiell gleiche Methode zur antimikrobiellen Impregnierung von Kathetern und anderen medizinischen Implantaten vor, bei denen ebenfalls ein antimikrobieller Wirkstoff in einem organischen Lösungsmittel gelöst wird (R. Darouche, I. Raad: Antimicobial impregnated catheters and other medical implants and method for impregnating catheters and other medical implants with an antimicrobial agent. 29.04.1997, US 5,624,704). Diese Lösung lässt man auf die zu behandelnde Oberfläche einwirken, wobei der Wirkstoff in das Material eindringt und sich dort ablagert..

Eine Alternative zu den bisher beschriebenen Systemen stellt eine von Lee beschriebene Methode zur Oberflächenbeschichtung mit kationischen Antibiotika dar (C. C. Lee: Coating medical devices with cationic antibiotics. 23.01.1990, US 4,895,566). Bei dieser Methode wird zuerst eine negativ geladene Heparinschicht auf die zu beschichtende Oberfläche aufgebracht und anschließend nach deren Anheftung lässt man an diese kationische Antibiotika anlagern. Eine ähnliche Lösung schlagen Greco et al. vor, bei der man zuerst eine Lösung von anionischen oberflächenaktiven Substanzen auf die zu beschichtende Oberfläche einwirken lässt (R. S. Greco, R. A. Harvey, S. Z. Trooskin: Drug bonded prosthesis and process for producing same. 07.11. 1989, US 4,879,135). Dabei adsorbieren die anionischen Moleküle auf der Oberfläche. Anschließend werden kationische Wirkstoffe, wie zum Beispiel Gentamicin, elektrostatisch angebunden. Es muss bei den beiden zuletzt zitierten Verfahren angemerkt werden, dass die Beladungsdichte mit Antibiotika pro Flächeneinheit sehr begrenzt ist und dass die Haftfestigkeit dieser Beschichtungen kritisch zu sehen ist.

Der vorliegenden Erfindung liegt die Aufgabe zugrunde, eine flexible Antibiotikum-/Antibiotika-Polymer-Kombination zu entwickeln, die unter physiologischen Bedingungen eine kontinuierliche Freisetzung von Antibiotika über einen Zeitraum von mehreren Tagen bis zu Wochen erlaubt und in der Human- und Veterinärmedizin eingesetzt werden kann. Diese Antibiotikum-/Antibiotika-Polymer-Kombination soll in einfacher Weise haftfest auf die Oberflächen von medizinischen Kunststoff- und Metallimplantaten aufgebracht werden können. Hierbei ist es insbesondere wichtig, dass die Beschichtung flexibel und elastisch ist und keine toxischen Komponenten abgegeben werden. Weiterhin soll die flexible Antibiotikum-/Antibiotikum-Polymer-Kombination zur Herstellung von antibiotischen Fäden, Folien und Formkörpern geeignet sein.

Die Aufgabe wird durch die Merkmale der unabhängigen Ansprüche gelöst. Vorteilhafte Ausgestaltungen ergeben sich aus den Unteransprüchen.

Der Erfindung liegt der überraschende Befund zugrunde, dass homogene Polymermischungen bestehend aus einem oder mehreren hydrophoben, nichtionischen Polymeren aus den Gruppen des Poly(vinylchlorids), des nachchlorierten Poly(vinylchlorids), des Poly(vinylidenchlorids), des Poly(vinylfluorids), des Poly(vinylidenfluorids und der Co-Polymere aus Vinylchlorid und einem oder mehreren nichtionischen Monomeren und aus einem oder mehreren hydrophilen Polymeren aus den Gruppen der Polyether, ein oder mehrere in Wasser gering lösliche antibiotische Salze aus den Gruppen der Aminoglykosid-Antibiotika, der Lincosamid-Antibiotika, der Tetracyclin-Antibiotika, der Glykopeptid-Antibiotika, der Chinolon-Antibiotika und des Chlorhexidins, suspendiert sind und diese Suspension Komposite bilden, die im wässrigen Milieu eine Wirkstoff-Freisetzung über einen Zeitraum von Tagen zeigen.

Die Aufgabe der Erfindung wird also dadurch gelöst, dass in einer homogenen Polymermischung, die aus einem oder mehreren hydrophoben, nichtionischen Polymeren aus den Gruppen des Poly(vinylchlorids), des nachchlorierten Poly(vinylchlorids), des Poly(vinylidenchlorids), des Poly(vinylfluorids), des Poly(vinylidenfluorids und der Co-Polymere aus Vinylchlorid und einem oder mehreren nichtionischen Monomeren und aus einem oder mehreren hydrophilen Polymeren aus den Gruppen der Polyether besteht, ein oder mehrere in Wasser gering lösliche antibiotische Salze aus den Gruppen der Aminoglykosid-Antibiotika, der Lincosamid-Antibiotika, der Tetracyclin-Antibiotika, der Glykopeptid-Antibiotika, der Chinolon-Antibiotika und des Chlorhexidins, ggf. einem in Wasser leichtlöslichem Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind und diese Suspension ein Komposit bildet.

Es ist deshalb erfindungsgemäß, dass in einer homogenen Polymermischung, die aus einem oder mehreren hydrophoben, nichtionischen Polymeren aus den Gruppen des Poly(vinylchlorids), des nachchlorierten Poly(vinylchlorids), des Poly(vinylidenchlorids), des Poly(vinylfluorids), des Poly(vinylidenfluorids und der Co-Polymere aus Vinylchlorid und einem oder mehreren nichtionischen Monomeren und aus einem oder mehreren hydrophilen Polymeren aus den Gruppen der Polyether besteht, ein oder mehrere Vertreter der in Wasser gering löslichen antibiotische Salze Gentamicin-Dodecylsulfat, Gentamicin-Dodecylsulfonat, Gentamicin-Laurat, Gentamicin-Decylsulfat, Amikacin-Dodecylsulfat, Amikacin-Dodecylsulfonat, Amikacin-Laurat, Kanamycin-Dodecylsulfat, Kanamycin-Dodecylsulfonat, Kanamycin-Laurat, Kanamycin-Myristat, Tobramycin-Dodecylsulfat, Tobramycin-Dodecylsulfonat, Tobramycin-Laurat, Tobramycin-Myristat, Vancomycin-Dodecylsulfat, Vancomycin-Laurat, Vancomycin-Myristat, Teicoplanin-Vancomycin, Clindamycin-Laurat, Tetracyclin-Dodecylsulfat, Tetracyclin-Laurat, Minocyclin-Dodecylsulfat, Minocyclin-Laurat, Oyxtetracyclin-Dodecylsulfat, Oxytetracyclin-Laurat, Rolitetracyclin-Laurat, Rolitetracyclin-Dodecylsulfat, Chlortetracyclin-Dodecylsulfat, Chlortetracyclin-Laurat, Ciprofloxacin-Laurat, Ciprofloxacin-Myristat, Moxifloxacin-Myristat, Chlorhexidin-Dodecylsulfat, Chlorhexidin-Laurat und Chlorhexidin-Caprinat, ggf. einem in Wasser leichtlöslichem Antibiotikum aus den Gruppen der Aminoglykosid-Antibiotika, der Lincosamid-Antibiotika, der β-lactam-Antibiotika und der Tetracyclin-Antibiotika, und ggf. ein oder mehrere organische Hilfsstoffe, suspendiert sind und diese Suspension ein Komposit bildet. Im Sinne der Erfindung ist auch die Verwendung von anderen in wässrigem Milieu gering löslichen Antibiotika und antimikrobiellen Chemotherapeutika.

Vorteilhaft ist, dass das Komposit aus einer fließfähigen Suspension, die aus einer homogenen Mischung von Cyclohexanon und oder Tetrahydrofuran und ggf. Weichemachem aus den Gruppen der Phthalsäureester, der Trimelitsäureester, der Phosphorsäureester, der Adipinsäureester, der Azelainsäureester, der Sebacinsäureester, einem oder mehreren hydrophoben, nichtionischen Polymeren aus den Gruppen des Poly(vinylchlorids) und der Co-Polymere aus Vinylchlorid und einem oder mehreren nichtionischen Monomeren und einem oder mehreren hydrophilen Polymeren aus den Gruppen der Polyether besteht, in der ein oder mehrere in Wasser gering lösliche antibiotischen Salze aus den Gruppen der Aminoglykosid-Antibiotika, der Lincosamid-Antibiotika, der Tetracyclin-Antibiotika, der Chinolon-Antibiotika und des Chlorhexidins, ggf. einem in Wasser leichtlöslichem Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind, durch Verdampfung von Cyclohexanon und oder Tetrahydrofuran, gebildet wird.

Vorteilhaft ist ebenfalls, dass Gemische aus Cyclohexanon und Tetrahydrofuran verwendet werden und dass auch andere zur Lösung von Polyvinylchlorid befähigten organischen Lösungsmittel und Lösungsmittelgemische verwendet werden.

Es ist auch vorteilhaft, dass das Komposit aus einer Schmelze gebildet wird, die aus einem oder mehreren hydrophoben, nichtionischen Polymeren aus den Gruppen des Poly(vinylchlorids) und oder der Co-Polymere aus Vinylchlorid und einem oder mehreren nichtionischen Monomeren und einem oder mehreren hydrophilen Polymeren aus den Gruppen der Polyether besteht und ggf. Weichemachem aus den Gruppen der Phthalsäureester, der Trimelitsäureester, der Phosphorsäureester, der Citronensäureester, der Weinsäureester, der Äpfelsäureester, der Fettsäureester, der Adipinsäureester, der Azelainsäureester, der Sebacinsäureester, in der ein oder mehrere in Wasser gering lösliche antibiotische Salze aus den Gruppen der Aminoglykosid-Antibiotika, der Lincosamid-Antibiotika, der Tetracyclin-Antibiotika, der Chinolon-Antibiotika und des Chlorhexidins, ggf. einem in Wasser leichtlöslichem Antibiotikum aus den Gruppen der Aminoglykosid-Antibiotika, der Lincosamid-Antibiotika und der Tetracyclin-Antibiotika, und ggf. ein oder mehrere organische Hilfsstoffe, suspendiert sind.

Weiterhin ist zweckmäßig, dass der Gehalt an hydrophilem Polymer in der homogenen Polymermischung zwischen 0,1 bis 60 Masseprozent beträgt.

Insbesondere ist es von Vorteil, dass Polyethylenglykol mit einer mittleren Molmasse im Bereich von 120 gmol⁻¹ bis 35000 gmol⁻¹ als Polyether verwendet wird.

Ebenfalls vorteilhaft ist, dass Polypropylenglykol mit einer mittleren Molmasse im Bereich von 200 gmol⁻¹ bis 35000 gmol⁻¹ als Polyether verwendet wird.

Zweckmäßigerweise wird Polyethylenglykol mit einer mittleren Molmasse im Bereich von 200 gmol⁻¹ bis 600 gmol⁻¹ als Polyether verwendet.

Vorteilhaft ist weiterhin, dass Co-Polymere des Vinylchlorids, die aus Vinylchlorid und den Co-Monomeren Vinylidenchlorid, Vinylfluorid, Vinylacetat, Acrylnitril, aliphatischen Arylsäureestern, aromatischen Acrylsäureestern, aliphatischen Methacrylsäureestern, aromatischen Methacrylsäureestern, Ethen, Propen, Butadien, Isopren, 2-Chlorbutadien und Isopropylen hergestellt sind, mit mittleren Molmassen von 20.000 gmol⁻¹ bis 2.000.000 gmol⁻¹ als hydrophobe Polymere verwendet werden.

Es ist des weiteren sinnvoll, dass Sulfonamide und/oder Antiphlogistika und/oder Anästhetika und/oder Vancomycinhydrochlorid als organische Hilfsstoffe bevorzugt werden.

Zweckmäßig ist auch, dass die fließfähige Suspension durch Spinnen, unter Verdampfung vonCyclohexanon und/oder Tetrahydrofuran, Komposite in Form von Fäden bildet, dass die fließfähige Suspension durch Gießen, unter Verdampfung Cyclohexanon und/oder Tetrahydrofuran Komposite in Form von Folien bildet oder dass die fließfähige Suspension durch Versprühen, unter Verdampfung von Cyclohexanon und/oder Tetrahydrofuran Komposite in Form von Pulvern und Granulaten bildet.

Vorteilhaft im Sinne der Erfindung ist auch, dass das Komposit durch Pressen, Strangpressen und Walzen zu Formkörpern und Folien geformt wird.

Zweckmäßig ist, dass die mit dem Komposit beschichteten Kunststoffschläuche, Kunststofffäden, Kunststofffolien, kugelförmigen Kunststoffkörper, walzenförmigen Kunststoffkörper und kettenförmigen Kunststoffkörper als medizinische Implantate verwendet werden.

Zweckmäßig ist weiterhin, dass Katheter, Tracheal-Kanülen und Schläuche zur intraperitonealen Ernährung mit dem Komposit beschichtet sind oder, dass implantierbare Metallplatten, Metallnägel, Metallschrauben mit dem Komposit beschichtet sind.

Weiterhin ist im Sinne der Erfindung, dass das Komposit zur Verklebung von medizinisch verwendbaren Kunststoffformkörpern, Kunststofffolien, Kunststofffäden, Metallplatten und Metallröhren verwendet wird.

Vorteilhaft ist, dass das Komposit als Bindemittel zur Herstellung von antibiotischen Formkörpern aus Kunststoffgranulaten, Kunststoffpulvern, resorbierbaren Glaspulvern, nichtresorbierbaren Glaspulvern und Quarz-Pulvern verwendet wird.

Vorteilhaft ist des weiteren, dass die fließfähige Suspension durch Tauchen, Sprühen, Streichen, Bürsten und Walzen auf die Oberfläche von Kunststoffen und/oder Metallen aufgebracht wird und durch Verdampfung von Cyclohexanon und/oder Tetrahydrofuran ein Komposit in Form einer Beschichtung bildet.

Ferner ist es sinnvoll, dass das Komposit als Beschichtung auf medizinisch verwendbaren Kunststofffäden, Kunststofffolien, Kunststoffschläuche, Kunststoffbeutel und Kunststoffflaschen aufgebracht wird.

Erfindungsgemäß bevorzugt ist, dass der Komposit als Beschichtung auf kugelförmige Formkörper, auf walzenförmige Formkörper und auf kettenförmige Formkörper aufgebracht wird, die aus Kunststoff und/oder Metall bestehen.

Ferner ist zweckmäßig, dass das Komposit als Beschichtung auf Formkörper, Folien und Fäden aus Poly(methacrylsäureester), Poly(acrylsäureester), Poly(methacrylsäuresster-co-acrylsäureester), Polyvinylchlorid, Polyvinylidenchlorid, Silikon, Polystyren und Polycarbonat aufgebracht wird.

Zweckmäßig ist ebenfalls, dass der Komposit als Bindemittel zur Herstellung von antibiotischen Laminaten verwendet wird.

Weiterhin ist vorteilhaft, dass das Komposit durch Sintern als Beschichtung auf die Oberfläche von Metallen und/oder Kunststoffen aufgebracht wird.

Die Erfindung soll an zwei Beispielen näher erläutert werden.

### Beispiel 1:

Es wird eine Lösung bestehend aus 1,50 g Polyvinylchlorid, 300 mg Polyethylenglykol 600 und 13,50 g Cyclohexanon hergestellt. Es werden dann separat 1,00 g Gentamicinsulfat (AK 628) in 1 ml dest. Wasser gelöst. Dann werden separat 0,50 g Natriumdodecylsulfat in 0,75 ml Wasser gelöst. In die Polyvinylchlorid-PEG600-Cyclohexanon-Lösung werden zuerst unter Rühren die wässrige Gentamicinsulfat-Lösung getropft und danach die wässrige Natriumdodecylsulfat-Lösung. In die entstandene Suspension wird ein 2,5 cm langes Stück eines üblichen PVC-Redonschlauches (d= 6mm) getaucht und anschließend bei Raumtemperatur getrocknet, wobei das Cyclohexanon abdampft. Es entsteht eine haftfeste Beschichtung auf der Schlauchoberfläche. Die Masse der Beschichtung beträgt 16 mg.

### Beispiel 2:

Es wird eine Lösung bestehend aus 1,50 g Polyvinylchlorid, 300 mg Polyethylenglykol 600 und 13,50 g Cyclohexanon hergestellt. Es werden dann 0,59 g Gentamicinsulfat (AK 628) in 1 ml dest. Wasser gelöst. Dann werden 0,25 g Natriumdodecylsulfat in 0,75 ml Wasser gelöst. In die Polyvinylchlorid-PEG600-Cyclohexanon-Lösung werden zuerst unter Rühren die wässrige Gentamicinsulfat-Lösung getropft und danach die wässrige Natriumdodecylsulfat-Lösung. Die entstandene Suspension wird mit einer üblichen Spritzpistole mit Druckluft auf ein 2,5 cm langes Stück eines üblichen PVC-Redonschlauches (d= 6 mm) aufgesprüht. Den besprühten PVC-Schlauch lässt man bei Raumtemperatur trocknen. Nach Abdunsten des Cyclohexanons ist eine fest auf der Schlauchoberfläche haftende Beschichtung entstanden. Die Masse der Beschichtung beträgt 18 mg.

### Gentamicin-Freisetzungsversuche

Die in den Beispielen 1 und 2 beschichteten Schlauchstücke wurden in Sörensen-Puffer pH 7,4 eingebracht und in dieser bei 37°C über einen Zeitraum von vier Wochen gelagert, um die retardierte Antibiotika-Freisetzung zu bestimmen. Die Probennahme erfolgte nach 1, 2, 3, 4, 5 und 6 Tagen Lagerungszeit. Die Antibiotika-Wertbestimmung wurde mit einem mikrobiellen Agardiffusionstest unter Verwendung von Bacillus subtilis ATCC 6633 als Testkeim durchgeführt.

**Tab.1:**

| Kumulierte Gentamicinsulfat-Freisetzung aus den beschichteten Schläuchen der Beispiele 1 und 2 in Abhängigkeit von der Lagerungszeit in physiologischer Kochsalzlösung bei 37°C. | | | | | | |
|---|---|---|---|---|---|---|
| Beispiele | kumulierte Gentamicinsulfat-Freisetzung [µg] (als Gentamicinsulfat AK628) | | | | | |
| | Lagerungszeit [d] | | | | | |
| | 1 | 2 | 3 | 4 | 5 | 6 |
| **1** | 1830 | 2170 | 2210 | 2230 | 2250 | 2260 |
| **2** | 1360 | 1440 | 1450 | 1460 | 1480 | 1490 |

## Patentansprüche

1. Flexible Antibiotikum-/Antibiotika-Polymer-Kombination für die Human- und Veterinärmedizin, **dadurch gekennzeichnet, dass** in einer homogenen Polymermischung, die aus einem oder mehreren hydrophoben, nichtionischen Polymeren aus den Gruppen des Poly(vinylchlorids), des nachchlorierten Poly(vinyl-chlorids), des Poly(vinylidenchlorids), des Poly(vinylfluorids), des Poly(vinylidenfluorids und der Co-Polymere aus Vinylchlorid und einem oder mehreren nichtionischen Monomeren und aus einem oder mehreren hydrophilen Polymeren aus den Gruppen der Polyether besteht, ein oder mehrere in Wasser gering lösliche antibiotische Salze aus den Gruppen der Aminoglykosid-Antibiotika, der Lincosamid-Antibiotika, der Tetracyclin-Antibiotika, der Glykopeptid-Antibiotika, der Chinolon-Antibiotika und des Chlorhexidins, ggf. ein in Wasser leichtlösliches Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind und diese Suspension ein Komposit bildet.

2. Flexible Antibiotikum-/Antibiotika-Polymer-Kombination für die Human- und Veterinärmedizin, **dadurch gekennzeichnet, dass** in einer homogenen Polymermischung, die aus einem oder mehreren hydrophoben, nichtionischen Polymeren aus den Gruppen des Poly(vinylchlorids), des nachchlorierten Poly(vinylchlorids), des Poly(vinylidenchlorids), des Poly(vinylfluorids), des Poly(vinylidenfluorids und der Co-Polymere aus Vinylchlorid und einem oder mehreren nichtionischen Monomeren und aus einem oder mehreren hydrophilen Polymeren aus den Gruppen der Polyether besteht, ein oder mehrere Vertreter der in Wasser gering löslichen antibiotische Salze Gentamicin-Dodecylsulfat, Gentamicin-Dodecylsulfonat, Gentamicin-Laurat, Gentamicin-Decylsulfat, Amikacin-Dodecylsulfat, Amikacin-Dodecylsulfonat, Amikacin-Laurat, Kanamycin-Dodecylsulfat, Kanamycin-Dodecylsulfonat, Kanamycin-Laurat, Kanamycin-Myristat, Tobramycin-Dodecylsulfat, Tobramycin-Dodecylsulfonat, Tobramycin-Laurat, Tobramycin-Myristat, Vancomycin-Dodecylsulfat, Vancomycin-Laurat, Vancomycin-Myristat, Teicoplanin-Vancomycin, Clindamycin-Laurat, Tetracyclin-Dodecylsulfat, Tetracyclin-Laurat, Minocyclin-Dodecylsulfat, Minocyclin-Laurat, Oyxtetracycin-Dodecylsulfat, Oxytetracyclin-Laurat, Rolitetracyclin-Laurat, Rolitetracyclin-Dodecylsulfat, Chlortetracyclin-Dodecylsulfat, Chlortetracyclin-Laurat, Ciprofloxacin-Laurat, Ciprofloxacin-Myristat, Moxifloxacin-Myristat, Chlorhexidin-Dodecyl-sulfat, Chlorhexidin-Laurat und Chlorhexidin-Caprinat, ggf. ein in Wasser leichtlösliches Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind und diese Suspension ein Komposit bildet.

3. Antibiotikum-/Antibiotika-Polymer-Kombination nach Anspruch 1 oder 2, **dadurch gekennzeichnet, dass** das Komposit aus einer fließfähigen Suspension, die aus einer homogenen Mischung von Cyclohexanon und/oder Tetrahydrofuran und ggf. Weichmachern aus den Gruppen der Phthalsäureester, der Trimelitsäureester, der Phosphorsäureester, der Adipinsäureester, der Azelainsäureester, der Sebacinsäureester, einem oder mehreren hydrophoben, nichtionischen Polymeren aus den Gruppen des Poly(vinylchlorids) und der Co-Polymere aus Vinylchlorid und einem oder mehreren nichtionischen Monomeren und einem oder mehreren hydrophilen Polymeren aus den Gruppen der Polyether besteht, in der ein oder mehrere in Wasser gering lösliche antibiotischen Salze aus den Gruppen der Amino-glykosid-Antibiotika, der Lincosamid-Antibiotika, der Tetracyclin-Antibiotika, der Chino-Ion-Antibiotika und des Chlorhexidins, ggf. ein in Wasser leichtlösliches Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind, durch Verdampfung von Cyclohexanon und/oder Tetrahydrofuran, gebildet wird.

4. Antibiotikum-/Antibiotika-Polymer-Kombination nach Anspruch 1 oder 2, **dadurch gekennzeichnet, dass** das Komposit aus einer Schmelze gebildet wird, die aus einem oder mehreren hydrophoben, nichtionischen Polymeren aus den Gruppen des Poly(vinylchlorids) und oder der Co-Polymere aus Vinylchlorid und einem oder mehreren nichtionischen Monomeren und einem oder mehreren hydrophilen Polymeren aus den Gruppen der Polyether besteht und ggf. Weichemachern aus den Gruppen der Phthalsäureester, der Trimelitsäureester, der Phosphorsäureester, der Citronensäureester, der Weinsäureester, der Äpfelsäureester, der Fettsäureester, der Adipinsäureester, der Azelainsäureester, der Sebacinsäureester, in der ein oder mehrere in Wasser gering lösliche antibiotische Salze aus den Gruppen der Aminoglykosid-Antibiotika, der Lincosamid-Antibiotika, der Tetracyclin-Antibiotika, der Chinolon-Antibiotika und des Chlorhexidins, ggf. ein in Wasser leichtlösliches Antibiotikum aus den Gruppen der Aminoglykosid-Antibiotika, der Lincosamid-Antibiotika und der Tetracyclin-Antibiotika, und ggf. ein oder mehrere organische Hilfsstoffe, suspendiert sind.

5. Antibiotikum-/Antibiotika-Polymer-Kombination nach einem der Ansprüche 1 bis 4, **dadurch gekennzeichnet, dass** der Gehalt an hydrophilem Polymer in der homogenen Polymermischung zwischen 0,1 bis 60 Masseprozent beträgt.

6. Antibiotikum-/Antibiotika-Polymer-Kombination nach einem der Ansprüche 1 bis 5, **dadurch gekennzeichnet, dass** Polyethylenglykol mit einer mittleren Molmasse im Bereich von 120 gmol⁻¹ bis 35.000 gmol⁻¹ als Polyether verwendet wird.

7. Antibiotikum-/Antibiotika-Polymer-Kombination nach einem der Ansprüche 1 bis 5, **dadurch gekennzeichnet, dass** Polypropylenglykol mit einer mittleren Molmasse im Bereich von 200 gmol⁻¹ bis 35.000 gmol⁻¹ als Polyether verwendet wird.

8. Antibiotikum-/Antibiotika-Polymer-Kombination nach einem der Ansprüche 1 bis 6, **dadurch gekennzeichnet, dass** Polyethylenglykol mit einer mittleren Molmasse im Bereich von 120 gmol⁻¹ bis 600 gmol⁻¹ als Polyether bevorzugt verwendet wird.

9. Antibiotikum-/Antibiotika-Polymer-Kombination nach einem der Ansprüche 1 bis 8, **dadurch gekennzeichnet, dass** Co-Polymere des Vinylchlorids, die aus Vinylchlorid und den CoMonomeren Vinylidenchlorid, Vinylfluorid, Vinylacetat, Acrylnitril, aliphatischen Arylsäureestern, aromatischen Acrylsäureestem, aliphatischen Methacrylsäureestern, aromatischen Methacrylsäureestern, Ethen, Propen, Butadien, Isopren, 2-Chlorbutadien und Isopropylen hergestellt sind, mit mittleren Molmassen von 20.000 gmol⁻¹ bis 2.000.000 gmol⁻¹ als hydrophobe Polymere verwendet werden.

10. Antibiotikum-/Antibiotika-Polymer-Kombination nach einem der Ansprüche 1 bis 9, **dadurch gekennzeichnet, dass** Sulfonamide und/oder Antiphlogistika und/oder Anästhetika als organische Hilfsstoffe verwendet werden.

11. Antibiotikum-/Antibiotika-Polymer-Kombination nach einem der Ansprüche 3 oder 5 bis 10, **dadurch gekennzeichnet, dass** die fließfähige Suspension durch Spinnen, unter Verdampfung von Cyclohexanon und/oder Tetrahydrofuran, Komposite in Form von Fäden bildet.

12. Antibiotikum-/Antibiotika-Polymer-Kombination nach einem der Ansprüche 3 oder 5 bis 10, **dadurch gekennzeichnet, dass** die fließfähige Suspension durch Gießen, unter Verdampfung von Cyclohexanon und/oder Tetrahydrofuran, Komposite in Form von Folien bildet.

13. Antibiotikum-/Antibiotika-Polymer-Kombination nach einem der Ansprüche 3 oder 5 bis 10, **dadurch gekennzeichnet, dass** die fließfähige Suspension durch Versprühen, unter Verdampfung von Cyclohexanon und/oder Tetrahydrofuran, Komposite in Form von Pulvern und Granulaten bildet.

14. Antibiotikum-/Antibiotika-Polymer-Kombination nach einem der Ansprüche 1 bis 13, **dadurch gekennzeichnet, dass** das Komposit durch Pressen, Strangpressen und Walzen zu Formkörpern, Beschichtungen und Folien geformt ist.

15. Körper zur Verwendung als medizinische Implantate, nämlich mit dem Komposit einer Antibiotikum-/Antibiotika-Polymer-Kombination nach einem der Ansprüche 1 bis 14 beschichtete Kunstoffschläuche, Kunstofffäden, Kunstofffolien, kugelförmigen Kunstoffkörper, walzenförmigen Kunstoffkörper und kettenförmigen Kunsstoffkörper.

16. Körper, nämlich mit dem Komposit einer Antibiotikum-/Antibiotika-Polymer-Kombination nach einem der Ansprüche 1 bis 14 beschichtete Katheter, Tracheal-Kanülen und Schläuche zur intraperitonealen Ernährung.

17. Körper, nämlich mit dem Komposit einer Antibiotikum-/Antibiotika-Polymer-Kombination nach einem der Ansprüche 1 bis 14 beschichtete implantierbare Metallplatten, Metallnägel, Metallschrauben.

18. Verwendung des Komposits einer Antibiotikum-/Antibiotika-Polymer-Kombination nach einem der Ansprüche 1 bis 10 zur Verklebung von medizinisch verwendbaren Kunststoffformkörpern, Kunststofffolien, Kunststofffäden, Metallplatten und Metallröhren .

19. Verwendung des Komposits einer Antibiotikum-/Antibiotika-Polymer-Kombination nach einem der Ansprüche 1 bis 10 als Bindemittel zur Herstellung von antibiotischen Formkörpern aus Kunststoffgranulaten, Kunststoffpulvern, resorbierbaren Glaspulvern, nichtresorbierbaren Glaspulvern und Quarz-Pulvern.

20. Verwendung des Komposits einer Antibiotikum-/Antibiotika-Polymer-Kombination nach einem der Ansprüchen 1 bis 10 als Bindemittel zur Herstellung von antibiotischen Laminaten.

21. Verfahren zur Beschichtung von Kunststoffen und/oder Metallen, wobei die fließfähige Suspension einer Antibiotikum-/Antibiotika-Polymer-Kombination nach einem der Ansprüche 3 oder 5 bis 10 durch Tauchen, Sprühen, Streichen, Bürsten und Walzen auf die Oberfläche von Kunststoffen und/oder Metallen aufgebracht wird und durch Verdampfung von Cyclohexanon einen Komposit in Form einer Beschichtung bildet.

22. Verwendung des Komposits einer Antibiotikum-/Antibiotika-Polymer-Kombination nach einem der Ansprüche 1 bis 10 als Beschichtung auf medizinisch verwendbaren Kunststofffäden, Kunststofffolien, Kunststoffschläuche, Kunststoffbeutel und Kunststoffflaschen.

23. Verwendung des Komposits einer Antibiotikum-/Antibiotika-Polymer-Kombination nach einem der Ansprüche 1 bis 10 als Beschichtung auf kugelförmigen Formkörpern, auf walzenförmigen Formkörpern und auf kettenförmigen Formkörpern, die aus Kunststoff und/oder Metall bestehen.

24. Verwendung des Komposits einer Antibiotikum-/Antibiotika-Polymer-Kombination nach einem der Ansprüche 1 bis 10 als Beschichtung auf Formkörpern, Folien und Fäden aus Poly(methacrylsäureester), Poly(acrylsäure-ester), Poly(meth-acryl-säureester-co-acrylsäureester), Polyvinylchlorid, Polyvinyliden-chlorid, Silikon, Polystyren und Polycarbonat.

25. Verwendung des Komposits einer Antibiotikum-/Antibiotika-Polymer-Kombination nach einem der Ansprüche 1 oder 2 oder 4 als gesinterte Beschichtung auf der Oberfläche von Metallen und/oder Kunststoffen.

## Claims

1. Flexible antibiotic/antibiotics polymer combination for human and veterinary medicine, **characterized in that** one or more slightly water-soluble antibiotic salts from the groups consisting of the aminoglycoside antibiotics, of the lincosamide antibiotics, of the tetracycline antibiotics, of the glycopeptide antibiotics, of the quinolone antibiotics and of chlorhexidine, optionally a freely water-soluble antibiotic from the groups consisting of the aminoglycoside antibiotics, of the lincosamide antibiotics, of the β-lactam antibiotics and of the tetracycline antibiotics, and optionally one or more organic excipients, are suspended in a homogeneous polymer blend which consists of one or more hydrophobic, nonionic polymers from the groups consisting of poly(vinyl chloride), of post-chlorinated poly(vinyl chloride), of poly(vinylidene chloride), of poly(vinyl fluoride), of poly(vinylidene fluoride) and of the copolymers of vinyl chloride and one or more nonionic monomers, and consists of one or more hydrophilic polymers from the groups consisting of the polyethers, and this suspension forms a composite.

2. Flexible antibiotic/antibiotics polymer combination for human and veterinary medicine, **characterized in that** one or more members of the slightly water-soluble antibiotic salts gentamicin dodecylsulphate, gentamicin dodecylsulphonate, gentamicin laurate, gentamicin decylsulphate, amikacin dodecylsulphate, amikacin dodecylsulphonate, amikacin laurate, canamycin dodecylsulphate, canamycin dodecylsulphonate, canamycin laurate, canamycin myristate, tobramycin dodecylsulphate, tobramycin dodecylsulphonate, tobramycin laurate, tobramycin myristate, vancomycin dodecylsulphate, vancomycin laurate, vancomycin myristate, teicoplanin-vancomycin, clindamycin laurate, tetracycline dodecylsulphate, tetracycline laurate, minocycline dodecylsulphate, minocycline laurate, oxytetracycline dodecylsulphate, oxytetracycline laurate, rolitetracycline laurate, rolitetracycline dodecylsulphate, chlorotetracycline dodecylsulphate, chlorotetracycline laurate, ciprofloxacin laurate, ciprofloxacin myristate, moxifloxacin myristate, chlorhexidine dodecylsulphate, chlorhexidine laurate and chlorhexidine caprinate, optionally a freely water-soluble antibiotic from the groups consisting of the aminoglycoside antibiotics, of the lincosamide antibiotics, of the β-lactam antibiotics and of the tetracycline antibiotics, and optionally one or more organic excipients, are suspended in a homogeneous polymer blend which consists of one or more hydrophobic, nonionic polymers from the groups consisting of poly(vinyl chloride), of post-chlorinated poly(vinyl chloride), of poly(vinylidene chloride), of poly(vinyl fluoride), of poly(vinylidene fluoride) and of the copolymers of vinyl chloride and one or more nonionic monomers, and consisting of one or more hydrophilic polymers from the groups consisting of the polyethers, and this suspension forms a composite.

3. Antibiotic/antibiotics polymer combination according to Claim 1 or 2; **characterized in that** the composite is formed from a flowable suspension which consists of a homogeneous mixture of cyclohexanone and/or tetrahydrofuran and optionally plasticizers from the groups consisting of the phthalic esters, of the trimellitic esters, of the phosphoric esters, of the adipic esters, of the azelaic esters and of the sebacic esters, one or more hydrophobic, nonionic polymers from the groups consisting of the poly(vinyl chloride) and of the copolymers of vinyl chloride and one or more nonionic monomers, and one or more hydrophilic polymers from the group consisting of the polyethers, in which one or more slightly water-soluble antibiotic salts from the groups consisting of the aminoglycoside antibiotics, of the lincosamide antibiotics, of the tetracycline antibiotics, of the quinolone antibiotics and of chlorhexidine, optionally a freely water-soluble antibiotic from the groups consisting of the aminoglycoside antibiotics, of the lincosamide antibiotics, of the β-lactam antibiotics and of the tetracycline antibiotics, and optionally one or more organic excipients, are suspended, by evaporation of cyclohexanone and/or tetrahydrofuran.

4. Antibiotic/antibiotics polymer combination according to Claim 1 or 2, **characterized in that** the composite is formed from a melt which consists of one or more hydrophobic, nonionic polymers from the groups consisting of poly(vinyl chloride) and/or of the copolymers of vinyl chloride and one or more nonionic monomers, and one or more hydrophilic polymers from the groups consisting of the polyethers, and optionally plasticizers from the groups consisting of the phthalic esters, of the trimellitic esters, of the phosphoric esters, of the citric esters, of the tartaric esters, of the malic esters, of the fatty acid esters, of the adipic esters, of the azelaic esters and of the sebacic esters, in which one or more slightly water-soluble antibiotic salts from the groups consisting of the aminoglycoside antibiotics, of the lincosamide antibiotics, of the tetracycline antibiotics, of the quinolone antibiotics and of chlorhexidine, optionally a freely water-soluble antibiotic from the groups consisting of the aminoglycoside antibiotics, of the lincosamide antibiotics and of the tetracycline antibiotics, and optionally one or more organic excipients, are suspended.

5. Antibiotic/antibiotics polymer combination according to any of Claims 1 to 4, **characterized in that** the content of hydrophilic polymer in the homogeneous polymer blend is between 0.1 and 60 percent by mass.

6. Antibiotic/antibiotics polymer combination according to any of Claims 1 to 5, **characterized in that** polyethylene glycol having an average molar mass in the range from 120 gmol⁻¹ to 35,000 gmol⁻¹ is used as the polyether.

7. Antibiotic/antibiotics polymer combination according to any of Claims 1 to 5, **characterized in that** polypropylene glycol having an average molar mass in the range from 200 gmol⁻¹ to 35,000 gmol⁻¹ is used as the polyether.

8. Antibiotic/antibiotics polymer combination according to any of Claims 1 to 6, **characterized in that** polyethylene glycol having an average molar mass in the range from 120 gmol⁻¹ to 600 gmol⁻¹ is preferably used as the polyether.

9. Antibiotic/antibiotics polymer combination according to any of Claims 1 to 8, **characterized in that** copolymers of vinyl chloride which are prepared from vinyl chloride and the comonomers vinylidene chloride, vinyl fluoride, vinyl acetate, acrylonitrile, aliphatic acrylic esters, aromatic acrylic esters, aliphatic methacrylic esters, aromatic methacrylic esters, ethene, propene, butadiene, isoprene, 2-chlorobutadiene and isopropylene, having average molar masses of from 20,000 gmol⁻¹ to 2,000,000 gmol⁻¹, are used as hydrophobic polymers.

10. Antibiotic/antibiotics polymer combination according to any of Claims 1 to 9, **characterized in that** sulphonamides and/or antiphlogistic agents and/or anaesthetics are used as organic excipients.

11. Antibiotic/antibiotics polymer combination according to any of Claims 3 or 5 to 10, **characterized in that** the flowable suspension forms composites in the form of filaments as a result of spinning, with evaporation of cyclohexanone and/or tetrahydrofuran.

12. Antibiotic/antibiotics polymer combination according to any of Claims 3 or 5 to 10, **characterized in that** the flowable suspension forms composites in the form of films as a result of casting, with evaporation of cyclohexanone and/or tetrahydrofuran.

13. Antibiotic/antibiotics polymer combination according to any of Claims 3 or 5 to 10, **characterized in that** the flowable suspension forms composites in the form of powders and granules as a result of spraying, with evaporation of cyclohexanone and/or tetrahydrofuran.

14. Antibiotic/antibiotics polymer combination according to any of Claims 1 to 13, **characterized in that** the composite is formed into mouldings, coatings and films by pressing, extrusion and rolling.

15. Bodies for use as medical implants, namely plastic tubes, plastic filaments, plastic films, spherical plastic bodies, roll-like plastic bodies and chain-like plastic bodies coated with the composite of an antibiotic/antibiotics polymer combination according to any of Claims 1 to 14.

16. Bodies, namely catheters, tracheal cannulae and tubes for intraperitoneal feeding which are coated with the composite of an antibiotic/antibiotics polymer combination according to any of Claims 1 to 14.

17. Bodies, namely implantable metal plates, metal pins and metal screws coated with the composite of an antibiotic/antibiotics polymer combination according to any of Claims 1 to 14.

18. Use of the composite of an antibiotic/antibiotics polymer combination according to any of Claims 1 to 10 for adhesive bonding of plastic mouldings, plastic films, plastic filaments, metal plates and metal tubes which can be used for medical purposes.

19. Use of the composite of an antibiotic/antibiotics polymer combination according to any of Claims 1 to 10 as a binder for the production of antibiotic mouldings from plastic granules, plastic powders, absorbable glass powders, nonabsorbable glass powders and quartz powders.

20. Use of the composite of an antibiotic/antibiotics polymer combination according to any of Claims 1 to 10 as a binder for the production of antibiotic laminates.

21. Process for the coating of plastics and/or metals, the flowable suspension of an antibiotic/antibiotics polymer combination according to any of Claims 3 or 5 to 10 being applied to the surface of plastics and/or metals by immersion, spraying, spreading, brushing and rolling, and a composite in the form of a coating being formed by evaporation of cyclohexanone.

22. Use of the composite of an antibiotic/antibiotics polymer combination according to any of Claims 1 to 10 as a coating on plastic filaments, plastic films, plastic tubes, plastic bags and plastic bottles which can be used for medical purposes.

23. Use of the composite of an antibiotic/antibiotics polymer combination according to any of Claims 1 to 10 as a coating on spherical mouldings, on roll-like mouldings and on chain-like mouldings which consist of plastic and/or metal.

24. Use of the composite of an antibiotic/antibiotics polymer combination according to any of Claims 1 to 10 as a coating on mouldings, films and filaments of poly(methacrylic esters), poly(acrylic esters), poly(methacrylic ester-co-acrylic ester), polyvinyl chloride, polyvinylidene chloride, silicone, polystyrene and polycarbonate.

25. Use of the composite of an antibiotic/antibiotics polymer combination according to any of Claims 1 and 2 and 4 as a sintered coating on the surface of metals and/or plastics.

## Revendications

1. Combinaison flexible antibiotique/antibiotiques-polymère pour la médecine humaine et vétérinaire, **caractérisée en ce qu'**un ou plusieurs sels antibiotiques peu solubles dans l'eau, appartenant aux groupes des antibiotiques aminoglycosidiques, des antibiotiques de type lincosamide, des antibiotiques de type tétracycline, des antibiotiques glycopeptidiques, des antibiotiques de type quinolone et de la chlorhexidine, éventuellement un antibiotique bien soluble dans l'eau appartenant aux groupes des antibiotiques aminoglycosidiques, des antibiotiques de type lincosamide, des antibiotiques de type β-lactame et des antibiotiques de type tétracycline, et éventuellement une ou plusieurs substances auxiliaires organiques, sont en suspension dans un mélange de polymères homogène, constitué d'un ou plusieurs polymères non ioniques hydrophobes appartenant aux groupes du poly(chlorure de vinyle), du poly(chlorure de vinyle) surchloré, du poly(chlorure de vinylidène), du poly(fluorure de vinyle), du poly(fluorure de vinylidène) et des copolymères de chlorure de vinyle et d'un ou plusieurs monomères non ioniques, et d'un ou plusieurs polymères hydrophiles du groupe des polyéthers, et cette suspension forme un composite.

2. Combinaison flexible antibiotique/antibiotiques-polymère pour la médecine humaine et vétérinaire, **caractérisée en ce qu'**un ou plusieurs représentants des sels antibiotiques peu solubles dans l'eau dodécylsulfate de gentamycine, dodécylsulfonate de gentamycine, laurate de gentamycine, décylsulfate de gentamycine, dodécylsulfate d'amikacine, dodécylsulfonate d'amikacine, laurate d'amikacine, dodécylsulfate de kanamycine, dodécylsulfonate de kanamycine, laurate de kanamycine, myristate de kanamycine, dodécylsulfate de tobramycine, dodécylsulfonate de tobramycine, laurate de tobramycine, myristate de tobramycine, dodécylsulfate de vancomycine, laurate de vancomycine, myristate de vancomycine, teicoplanine-vancomycine, laurate de clindamycine, dodécylsulfate de tétracycline, laurate de tétracycline, dodécylsulfate de minocycline, laurate de minocycline, dodécylsulfate d'oxytétracycline, laurate d'oxytétracycline, laurate de rolitétracycline, dodécylsezlfate de rolitétracycline, dodécylsulfate de chlortétracycline, laurate de chlortétracycline, laurate de ciprofloxacine, myristate de ciprofloxacine, myristate de moxifloxacine, dodécylsulfate de chlorhexidine, laurate de chlorhexidine et caprate de chlorhexidine, éventuellement un antibiotique bien soluble dans l'eau appartenant aux groupes des antibiotiques aminoglycosidiques, des antibiotiques de type lincosamide, des antibiotiques de type β-lactame et des antibiotiques de type tétracycline, et éventuellement une ou plusieurs substances auxiliaires organiques, sont en suspension dans un mélange de polymères homogène, constitué d'un ou plusieurs polymères non ioniques hydrophobes appartenant aux groupes du poly(chlorure de vinyle), du poly(chlorure de vinyle) surchloré, du poly(chlorure de vinylidène), du poly(fluorure de vinyle), du poly(fluorure de vinylidène) et des copolymères de chlorure de vinyle et d'un ou plusieurs monomères non ioniques, et d'un ou plusieurs polymères hydrophiles du groupe des polyéthers, et cette suspension forme un composite.

3. Combinaison antibiotique/antibiotiques-polymère selon la revendication 1 ou 2, **caractérisée en ce que** le composite est formé à partir d'une suspension fluide, qui est constituée d'un mélange homogène de cyclohexanone et/ou de tétrahydrofurane et éventuellement de plastifiants appartenant aux groupes des esters de l'acide phtalique, des esters de l'acide trimellitique, des esters de l'acide phosphorique, des esters de l'acide adipique, des esters de l'acide azélaïque, des esters de l'acide sébacique, d'un ou plusieurs polymères non ioniques hydrophobes appartenant aux groupes du poly(chlorure de vinyle) et des copolymères de chlorure de vinyle et d'un ou plusieurs monomères non ioniques, et d'un ou plusieurs polymères hydrophiles du groupe des polyéthers, dans lequel un ou plusieurs sels antibiotiques peu solubles dans l'eau, appartenant aux groupes des antibiotiques aminoglycosidiques, des antibiotiques de type lincosamide, des antibiotiques de type tétracycline, des antibiotiques de type quinolone et de la chlorhexidine, éventuellement un antibiotique bien soluble dans l'eau appartenant aux groupes des antibiotiques aminoglycosidiques, des antibiotiques de type lincosamide, des antibiotiques de type β-lactame et des antibiotiques de type tétracycline, et éventuellement une ou plusieurs substances auxiliaires organiques, sont en suspension, par évaporation de la cyclohexanone et/ou du tétrahydrofurane.

4. Combinaison antibiotique/antibiotiques-polymère selon la revendication 1 ou 2, **caractérisée en ce que** le composite est formé à partir d'un mélange fondu, qui est constitué d'un ou plusieurs polymères non ioniques hydrophobes appartenant aux groupes du poly(chlorure de vinyle) et/ou des copolymères de chlorure de vinyle et d'un ou plusieurs monomères non ioniques, et d'un ou plusieurs polymères hydrophiles du groupe des polyéthers, et éventuellement de plastifiants appartenant aux groupes des esters de l'acide phtalique, des esters de l'acide trimellitique, des esters de l'acide phosphorique, des esters de l'acide citrique, des esters de l'acide tartrique, des esters de l'acide malique, des esters d'acides gras, des esters de l'acide adipique, des esters de l'acide azélaïque, des esters de l'acide sébacique, dans lequel un ou plusieurs sels antibiotiques peu solubles dans l'eau, appartenant aux groupes des antibiotiques aminoglycosidiques, des antibiotiques de type lincosamide, des antibiotiques de type tétracycline, des antibiotiques de type quinolone et de la chlorhexidine, éventuellement un antibiotique bien soluble dans l'eau appartenant aux groupes des antibiotiques aminoglycosidiques, des antibiotiques de type lincosamide et des antibiotiques de type tétracycline, et éventuellement une ou plusieurs substances auxiliaires organiques, sont en suspension.

5. Combinaison antibiotique/antibiotiques-polymère selon l'une des revendications 1 à 4, **caractérisée en ce que** la teneur en polymère hydrophile du mélange de polymères homogène est comprise entre 0,1 et 60 % en masse.

6. Combinaison antibiotique/antibiotiques-polymère selon l'une des revendications 1 à 5, **caractérisée en ce que** l'on utilise du polyéthylèneglycol ayant une masse molaire moyenne dans le domaine de 120 gmol⁻¹ à 35 000 gmol⁻¹ comme polyéther.

7. Combinaison antibiotique/antibiotiques-polymère selon l'une des revendications 1 à 5, **caractérisée en ce que** l'on utilise du polypropylèneglycol ayant une masse molaire moyenne dans le domaine de 200 gmol⁻¹ à 35 000 gmol⁻¹ comme polyéther.

8. Combinaison antibiotique/antibiotiques-polymère selon l'une des revendications 1 à 6, **caractérisée en ce que** l'on utilise de préférence du polyéthylèneglycol ayant une masse molaire moyenne dans le domaine de 120 gmol⁻¹ à 600 gmol⁻¹ comme polyéther.

9. Combinaison antibiotique/antibiotiques-polymère selon l'une des revendications 1 à 8, **caractérisée en ce que** l'on utilise comme polymères hydrophobes des copolymères du chlorure de vinyle, qui sont préparés à partir de chlorure de vinyle et des comonomères chlorure de vinylidène, fluorure de vinyle, acétate de vinyle, acrylonitrile, esters aliphatiques de l'acide acrylique, esters aromatiques de l'acide acrylique, esters aliphatiques de l'acide méthacrylique, esters aromatiques de l'acide méthacrylique, éthène, propène, butadiène, isoprène, 2-chlorobutadiène et isopropylène, avec des masses molaires moyennes de 20 000 gmol⁻¹ à 2 000 000 gmol⁻¹.

10. Combinaison antibiotique/antibiotiques-polymère selon l'une des revendications 1 à 9, **caractérisée en ce que** l'on utilise des sulfonamides et/ou des antiphlogistiques et/ou des anesthésiques comme substances auxiliaires organiques.

11. Combinaison antibiotique/antibiotiques-polymère selon l'une des revendications 3 ou 5 à 10, **caractérisée en ce que** la suspension fluide forme des composites sous forme de fils par filage, avec évaporation de la cyclohexanone et/ou du tétrahydrofurane.

12. Combinaison antibiotique/antibiotiques-polymère selon l'une des revendications 3 ou 5 à 10, **caractérisée en ce que** la suspension fluide forme des composites sous forme de feuilles par coulée, avec évaporation de la cyclohexanone et/ou du tétrahydrofurane.

13. Combinaison antibiotique/antibiotiques-polymère selon l'une des revendications 3 ou 5 à 10, **caractérisée en ce que** la suspension fluide forme des composites sous forme de poudres et de granulés par pulvérisation, avec évaporation de la cyclohexanone et/ou du tétrahydrafurane.

14. Combinaison antibiotique/antibiotiques-polymère selon l'une des revendications 1 à 13, **caractérisée en ce que** le composite est moulé en corps moulés, revêtements et feuilles par compression, par extrusion et par laminage.

15. Corps, c'est-à-dire tuyaux en matière plastique, fils de matière plastique, feuilles de matière plastique, corps sphériques en matière plastique, corps cylindriques en matière plastique et corps en matière plastique en forme de chaînes, revêtus du composite d'une combinaison antibiotique/antibiotiques-polymère selon l'une des revendications 1 à 14, à utiliser comme implants médicaux.

16. Corps, c'est-à-dire cathéters, canules trachéales et tuyaux pour l'alimentation parentérale, revêtus du composite d'une combinaison antibiotique/antibiotiques-polymère selon l'une des revendications 1 à 14.

17. Corps, c'est-à-dire plaques métalliques, aiguilles métalliques, vis métalliques, revêtus du composite d'une combinaison antibiotique/antibiotiques-polymère selon l'une des revendications 1 à 14.

18. Utilisation du composite d'une combinaison antibiotique/antibiotiques-polymère selon l'une des revendications 1 à 10 pour le collage de corps moulés en matière plastique, de feuilles de matière plastique, de fils de matière plastique, de plaques métalliques et de tubes métalliques utilisables en médecine.

19. Utilisation du composite d'une combinaison antibiotique/antibiotiques-polymère selon l'une des revendications 1 à 10 comme liant pour la préparation de corps moulés antibiotiques à partir de granulés de matière plastique, de poudres de matière plastique, de poudres de verre résorbables, de poudres de verre non résorbables et de poudres de quartz.

20. Utilisation du composite d'une combinaison antibiotique/antibiotiques-polymère selon l'une des revendications 1 à 10 comme liant pour la préparation de stratifiés antibiotiques.

21. Procédé de revêtement de matières plastiques et/ou de métaux, dans lequel la suspension fluide d'une combinaison antibiotique/antibiotiques-polymère selon l'une des revendications 3 ou 5 à 10 est appliquée sur la surface de matières plastiques et/ou de métaux par immersion, pulvérisation, enduction, brossage et laminage, et forme un composite sous forme d'un revêtement par évaporation de la cyclohexanone.

22. Utilisation du composite d'une combinaison antibiotique/antibiotiques-polymère selon l'une des revendications 1 à 10 comme revêtement sur des fils de matière plastique, des feuilles de matière plastique, des tuyaux en matière plastique, des sachets en matière plastique et dse flacons en matière plastique utilisables en médecine.

23. Utilisation du composite d'une combinaison antibiotique/antibiotiques-polymère selon l'une des revendications 1 à 10 comme revêtement sur des corps moulés sphériques, des corps moulés cylindriques et des corps moulés en forme de chaîne constitués de matière plastique et/ou de métal.

24. Utilisation du composite d'une combinaison antibiotique/antibiotiques-polymère selon l'une des revendications 1 à 10 comme revêtement sur des corps moulés, des feuilles et des fils en poly(ester de l'acide méthacrylique), poly(ester de l'acide acrylique), poly(ester de l'acide méthacrylique-co-ester de l'acide acrylique), poly(chlorure de vinyle), poly(chlorure de vinylidène), silicone, polystyrène et polycarbonate.

25. Utilisation du composite d'une combinaison antibiotique/antibiotiques-polymère selon l'une des revendications 1 ou 2 ou 4 comme revêtement fritté sur la surface de métaux et/ou de matières plastiques.
